# EUROPEAN PATENT SPECIFICATION

(11) **EP 4 138 549 B1**
(45) Date of publication and mention of the grant of the patent: **12.03.2025**
(21) Application number: 21719175.8
(22) Date of filing: 21.04.2021
(51) Int. Cl.: A01K 67/0271, A61K 39/00, C07K 14/725

(54) **CHIMERIC GALLINACEOUS BIRD EMBRYO MODEL FOR STUDYING CANCER IMMUNOTHERAPY**
CHIMÄRES GALLINACES VOGELEMBRYOMODELL ZUR UNTERSUCHUNG DER KREBSIMMUNTHERAPIE
MODÈLE D'EMBRYON D'OISEAUX GALLINÉS CHIMÉRIQUES POUR L'ÉTUDE DE L'IMMUNOTHÉRAPIE ANTICANCÉREUSE

(30) Priority: 23.04.2020 EP 20305399
(43) Date of publication of application: 01.03.2023
(73) Proprietor: Oncofactory, 69100 Villeurbanne (FR); Centre National de la Recherche Scientifique (CNRS), 75016 Paris (FR); Université Claude Bernard Lyon 1, 69100 Villeurbanne (FR); Institut National de la Santé et de la Recherche Médicale (INSERM), 75013 Paris (FR)
(72) Inventor: CASTELLANI, Valérie, 69500 Bron (FR); DELLOYE-BOURGEOIS, Céline, 69740 Genas (FR); COSTECHAREYRE, Clélia, 69008 Lyon (FR); JAROSSON, Loraine, 69002 Lyon (FR)
(74) Representative: Regimbeau
(86) International application number: PCT/EP2021/060385
(87) International publication number: WO 2021/214137

(56) References cited:
- WO-A1-2017/079646
- WO-A1-2017/103025
- WO-A1-2020/075168
- CHRISTIAN BUSCH ET AL: "The Chick Embryo as an Experimental System for Melanoma Cell Invasion", PLOS ONE, vol. 8, no. 1, 14 January 2013 (2013-01-14), pages e53970, XP055714601, DOI: 10.1371/journal.pone.0053970
- I. R. CORDEIRO ET AL: "Chick embryo xenograft model reveals a novel perineural niche for human adipose-derived stromal cells", BIOLOGY OPEN, vol. 4, no. 9, 28 August 2015 (2015-08-28), pages 1180 - 1193, XP055714315, DOI: 10.1242/bio.010256
- MILIOTOU N. ANDROULLA ET AL: "CAR T-cell Therapy: A New Era in Cancer Immunotherapy", CURRENT PHARMACEUTICAL BIOTECHNOLOGY, vol. 19, no. 1, 31 May 2018 (2018-05-31), NL, pages 5 - 18, XP055560547, ISSN: 1389-2010, DOI: 10.2174/1389201019666180418095526
- ELIZABETH LOUISE SIEGLER ET AL: "Preclinical Models in Chimeric Antigen Receptor-Engineered T-Cell Therapy", HUMAN GENE THERAPY, vol. 29, no. 5, May 2018 (2018-05-01), GB, pages 534 - 546, XP055714818, ISSN: 1043-0342, DOI: 10.1089/hum.2017.243
- JEAN-LUC BOULLAND ET AL: "Xenotransplantation of Human Stem Cells into the Chicken Embryo", JOURNAL OF VISUALIZED EXPERIMENTS, no. 41, 11 July 2010 (2010-07-11), XP055714251, DOI: 10.3791/2071
- PARK T S ET AL: "Human embryonic stem cell-derived hematoendothelial progenitors engraft chicken embryos", EXPERIMENTAL HEMATOLOGY, ELSEVIER INC, US, vol. 37, no. 1, January 2009 (2009-01-01), pages 31 - 41, XP025801800, ISSN: 0301-472X, [retrieved on 20081026], DOI: 10.1016/J.EXPHEM.2008.08.007
- ANKRI C ET AL: "Human T cells engineered to express a programmed death 1/28 costimulatory retargeting molecule display enhanced antitumor activity", THE JOURNAL OF IMMUNOLOGY, WILLIAMS & WILKINS CO, US, vol. 191, no. 8, 1 October 2013 (2013-10-01), pages 4121 - 4129, XP002743490, ISSN: 0022-1767, [retrieved on 20130911], DOI: 10.4049/JIMMUNOL.1203085

## Description

### FIELD OF THE INVENTION

The present invention relates to an animal model for the study of cancer cells, in particular of the relation of cancer cells and immune cells. The animal model of the invention is particularly relevant for the study of immunotherapy therapeutic strategies.

### INTRODUCTION

The modelling of human cancers in laboratory animals is a central issue in preclinical tests accompanying the development of new anticancer therapies. The major criteria for the animal models developed to that effect are the model's reliability and its speed and cost of execution.

The animal models which have been developed for tumor studies and are in current use are chiefly mouse models. Preparation of these models involves a relatively long execution time and high costs. In addition, certain types of cancer cells cannot be implanted into mouse animal models.

The gallinaceous bird embryo, particularly the chick or quail embryo, is an attractive model for performing *ex vivo* experiments, in particular for the study of embryonic development and for xenotransplantation experiments. It is indeed inexpensive, very accessible and easy to handle. Furthermore, approval of studies by an Ethics Committee is not required, since the use of bird embryos during the first two-thirds of embryonic development is not considered as "animal experimentation" (European Directive 2010/63/EU). It is a model of choice for the study of cell proliferation, differentiation and migration. This animal model can also be used to study tumors.

A classic study model using the gallinaceous bird embryo is the grafting of exogenous cells at the extraembryonic structures, more precisely onto the chorioallantoic membrane (CAM). Tumor cells are implanted on the chick embryo membrane. After incubation for about 2 days, a tumor forms. This tumor takes advantage of the embryonic membrane vasculature, which is particularly developed, in order to grow. Such a system has made it possible to reproduce *in vivo* human tumors, notably glioblastoma, having cellular and molecular features similar to those observed in the tumors *in vivo.*

This chorioallantoic membrane graft model is also widely used to screen therapeutic molecules, notably molecules for inhibiting tumor angiogenesis. For example, WO 2015/074050 and US 2013/0171680 describe the xenograft of malignant human hematopoietic cells into extraembryonic structures: cancer cells are injected into the amniotic sac, the yolk sac, or the blood vessels of the CAM.

The international application WO 2020/075168 describes the graft, on the CAM, of cancer cells and immune cells, both dispersed in hydrogels.

Park *et al.* (2009) describe the introduction of human hematoendothelial progenitors into the yolk sac of chicken embryos; these progenitor cells were able to engraft in hematopoietic organs of the chicken embryo.

Some authors have reported the introduction of cancer cells into blood vessels or lumen of neural tubes of the chicken embryo.

Carter *et al.* (2012) report the injection of human neuroblastoma cells into the blood vessels of a chick embryo. Boulland *et al.* (2010) disclose the injection of human stem cells, in extraembryonic blood vessels or in lesions made in the neural tube.

Busch *et al.* (2012) describe the injection of melanoma cells into the lumen of the rhombencephalic vesicle of the brain of chicken embryos.

Jayachandran *et al.* (2015) teach the injection of melanoma cells into the lumen of the neural tube of chicken embryos, while Joel *et al.* (2013) teaches the injection of human gliobastoma cells into the same location.

None of these technologies allows the implantation of tumoral cells into the tissues of the embryo and therefore the formation of tumors. On the contrary, cancer cells introduced to these locations tend to reprogram into a more benign phenotype, and to disappear quickly.

Both patent applications WO 2016/05398 and WO 2017/103025 describe a novel animal model where cancer cells are grafted within the tissues of a gallinaceous bird embryo, and not into the extraembryonic structures, neither injected into vessels or lumens of tubes of said embryos. Advantageously, the introduction of cancer cells into the tissues of the embryo allow cancer cells to follow pathways of migration, guided by hormonal signals circulating into the embryo. In this animal model, cancer cells migrate to specific tissues in accordance with their nature, and then implant and form tumors within said tissues.

The present invention relates to the development of another animal model for the study of cancers, designed specifically for the study of interactions of cancer cells and immune cells. This animal model is particularly useful for the screening of immunotherapeutic drugs and/or cells.

### Immunotherapy therapeutic strategies

Immunotherapy can be defined as a type of treatment that helps/boosts the immune system of cancer patients to fight cancer. Indeed, as part of its normal function, the immune system is able to detect and destroy abnormal cells, in particular tumoral cells, and most likely prevents the growth of many cancers that are early-destroyed and therefore never detected. In the case of diagnosed tumors, immune cells are found in and around said tumors. Presence of these immune cells, called tumor-infiltrating lymphocytes or TILs, are a sign that the immune system is responding to the tumor. Generally, presence of lymphocytes into or around the tumor is a marker of good prognosis.

Unfortunately, it has been established that some tumors have found a way to escape the immune system, which is their mean to avoid destruction.

For example, cancer cells may:
- Have genetic changes that make them less visible to the immune system;
- Have proteins on their surface that turn off immune cells; and/or
- Change the normal cells around the tumor so they interfere with how the immune system responds to the cancer cells.

The main goal of immunotherapy is to restore interactions between immune system and cancer cells. Several types of immunotherapy are used to treat cancer, to stimulate the detection and/or destruction of the tumor cells by the cancer patient own's immune system. These include:
- Immune checkpoint inhibitors, which are drugs that block immune "checkpoints". These checkpoints are a normal part of the immune system that keep immune responses from being too strong. By blocking them, these drugs allow immune cells to respond more strongly to cancer.
- Immune system modulators, which enhance the body's immune response against cancer.
- Monoclonal antibodies, designed to bind to specific targets on cancer cells. Monoclonal antibodies are able to "mark" cancer cells so that they will be better seen and destroyed by the immune system.

The use of monoclonal antibodies in cancer therapy was first introduced in 1997 with rituximab, an anti-CD20 antibody for treatment of B cell lymphoma.
- T-cell transfer therapy, a treatment that restores and/or boosts the natural ability of T cells to fight cancer. T-cell transfer therapy may also be called adoptive cell therapy, adoptive immunotherapy, or immune cell therapy.

In 1996, it was shown that blocking CTLA-4, a key element of T-cell exhaustion, led to the rejection of rectal carcinoma in mice (Leach *et al.,* 1996).

### T cell exhaustion

T lymphocytes that are present in the tumor microenvironment (TME, *i.e.* cancer cells, inflammatory cells and suppressive cytokines) are generally "exhausted". T cell exhaustion is characterized by a loss of functionality and expression of inhibitory molecules. Exhausted T cells lack the capability to secrete effector molecules such as IL-2, IFN-γ, TNF-α and Granzyme B, and express inhibitory receptors such as PD1, TIM-3, LAG3, and CTLA-4 (McLane *et al.,* 2019).

The therapeutic strategy aiming to reverse the exhausted phenotype of T cells has been a point of focus for the last decade. Significant progress in the management of multiple types of cancers has been achieved. In particular, the clinical data of αPD1 (Pembrolizumab, Nivolumab) and/or αCTLA-4 (Ipilimumab) indicates overwhelming success. Without surprise, the expression of PDL1 on tumor tissue, the ligand of PD1, is associated with better response rates to these immunotherapeutic drugs.

### Targeting specific epitopes - CAR-T

Another shift of paradigm in immunotherapy has been the emergence of chimeric antigen receptor-T cells designated as CAR-T cells. CAR-T cells are autologous T cells genetically modified to express a chimeric receptor targeting a specific epitope. The specific portion of the CAR is connected, through a transmembrane domain, to signal transduction domains of CD28, 41BB and CD3ξ, allowing for efficient activation and optimal survival of T cells upon adoptive transfer (Feins *et al.,* 2019).

Remarkable success has been achieved in B cell leukemia with the use of CD19 specific CAR-T cells. However, the CAR-T cells strategy for solid tumors seems to be much less efficient, perhaps in consequence of multifactorial reasons such as lack of accessibility of the tumor microenvironment (TME), the immunosuppressive capacities of said TME, and the Tumor Associated Antigen immunogenecity/heterogeneity. Nevertheless, new constructs of CAR-T cells for solid tumors are still under investigation with several new targets, different addressing modes to the TME and various strategies for CAR-T cells persistance/survival being tested (Martinez & Moon, 2019).

Furthermore, another point of focus is the development of allogeneic CAR-T cells, issued from donors. Indeed, this technology has many potential advantages over autologous approaches, such as the immediate availability of cryopreserved batches for patient treatment, the possible standardization of the CAR-T cell product, time for multiple cell modifications, redosing or combination of CAR-T cells directed against different targets, and, last but not least, decreased cost using an industrialized process. Therefore, the development of allogeneic CAR-T cells is an active area of research.

### Models for studying immunotherapy

The emergence of new therapeutic strategies and to a greater degree, the apparent heterogeneity of responses to the immunomodulatory interventions, necessitate practical models in order to categorize patients prior starting treatments.

From a clinical point of view, the scoring of patients can be based on the expression of biomarkers such as the PD1/PDL1 axis, genetic/transcriptomic screening, the presence of appropriate TILs and/or the capability for these TILs to accomplish effector functions in the context of the tumor, etc.

Further, several mice models of patient-derived xenograft (PDX) have been developed. The general strategy is to graft patient-derived tumor tissue, and eventually co-graft patient-derived immune cells into immunodeficient mice recipients.

However, this approach is time consuming, expensive and not reliable since the engraftment success can be as low as 10%. Efforts and pecuniary investments for the generation of such animal models is incompatible with the clinical context (Jung et al., 2018).

In view of this situation, novel animal models for studying *in vivo* interactions of cancer cells and immune cells, preferentially for each and every patient, are actively searched. Preclinical models for testing CAR-T engineered cells are also needed (Siegler and Wang, 2018).

In this context, the present invention proposes a novel animal model, consisting in a chimeric gallinaceous bird embryo, that is particularly useful for the screening of immunotherapeutic drugs and/or cells, and for the categorization of patients.

### SUMMARY OF THE INVENTION

The present invention concerns a chimeric gallinaceous bird embryo comprising both types of exogenous cells:
a. At least one population of cancer cells, and
b. At least one population of immune cells,

wherein said exogenous cancer cells are present in at least one tissue of said embryo, and said exogenous immune cells are present in at least one tissue of said embryo and/or circulate in the blood vessels of said embryo,
wherein the gallinaceous bird embryo is from a chicken (*Gallus gallus*) or from a quail (*Coturnix japonica*)*,* and
wherein said embryo tissue designates any tissue of the embryo with the exception of the extra-embryonic annexes and the lumen of tubes and vessels.

The invention also relates to a process for obtaining a chimeric gallinaceous bird embryo as described above, i.e. comprising both types of exogenous cells:
a. At least one population of cancer cells, and
b. At least one population of immune cells,

comprising the following steps:
   - Introducing both populations of immune cells and cancer cells into a gallinaceous bird embryo, wherein the gallinaceous bird embryo is at a developmental stage between stage HH10 and stage HH30, and
   - incubating the chimeric embryo for at least 6 hours at a temperature comprised between 35° C and 42° C,
wherein said exogenous cancer cells are introduced into at least one tissue of said embryo, and said exogenous immune cells are introduced in at least one tissue of said embryo and/or in the blood vessels of said embryo,
wherein the gallinaceous bird embryo is from a chicken (*Gallus gallus*) or from a quail (*Coturnix japonica*)*,* and
wherein said embryo tissue designates any tissue of the embryo with the exception of the extra-embryonic annexes and the lumen of tubes and vessels.

At least two implementations of this process are hereby considered:
1) a process comprising the following steps:
   - Co-grafting both populations of immune cells and cancer cells within at least one tissue of a gallinaceous bird embryo at a developmental stage between stage HH10 and stage HH30, and
   - incubating the chimeric embryo for at least 6 hours at a temperature comprised between 35° C and 42°C,
      and
2) a process comprising the following steps:
   - Grafting the at least one population of cancer cells within at least one tissue of a gallinaceous bird embryo at a developmental stage between stage HH10 and stage HH30,
   - Injecting said at least one population of immune cells into the blood vessels of said embryo, and
   - incubating the grafted embryo for at least 6 hours at a temperature comprised between 35° C and 42°C.

The present invention also relates to an animal model for the study of human cancers consisting in a chimeric gallinaceous bird embryo as described above, or as obtained by the process previously presented.

The present invention also concerns the use of such animal model for screening at least one anti-cancer therapy chosen among the following group: immunotherapy, chemotherapy, targeted therapy, hormonotherapy, anti-cancer drugs and therapeutic enzymes.

The present invention also concerns the use of said animal model for assessing the activity of genetically engineered CAR-T cells.

### BRIEF DESCRIPTION OF THE FIGURES

**Figure 1****:** Pictures of labeled human PBMCs two days post-injection (1A) or post-grafting (1B) in an avian embryo. Fluorescent cells (in white) are immune cells that have settled in the embryonic tissues.
**Figure 2****:** Analysis of human PBMCs injected in the avian embryo by FACS, two days after injection. CFSE: Carboxyfluorescein succinimidyl ester; FSC: Forward scatter; SSC: Side scatter.
**Figure 3****:** Survival of PBMCs after injection into the chick embryo. PBMCs have been injected at different doses (represented on the abscissa axis); the percentage of cell survival is presented on the ordinate axis. Each bullet (Triangles, squares, lozenges, circles) represents an independent experiment with a determined number of injected PBMCs.
**Figure 4****:** Analyse of PBMC subpopulation post graft by flow cytometry
   Determination of the relative proportions of subpopulations of immune cells after grafting of PBMCs. PBMCs: cells prior to the grafting. AVI-PBMCs: PBMC cells grafted and analyzed after Percoll isolation from collagenase-treated embryos (after 48 hours of incubation of the embryo).
   (4A) CD19+ and CD3+ cells are quantified. Values are expressed in ratios of total identified lymphocytes. Ratio of CD3+/CD4+ and CD3+/CD8+ are also illustrated.
   (4B) Comparison by FACS analysis of subpopulations of immune cells (over alive CD45+ population) in the samples before the grafting (PBMCs) and two days after grafting in the avian embryo (AVI-PBMCs).
   (4C) Comparison by FACS analysis of markers of immune cells activation and exhaustion (over alive CD45+CD3+ population), before the grafting (PBMCs) and two days after grafting in the avian embryo (AVI-PBMC). Markers of activation: human CD69 and CD25; Markers of exhaustion: human TIM-3 and PD-1.

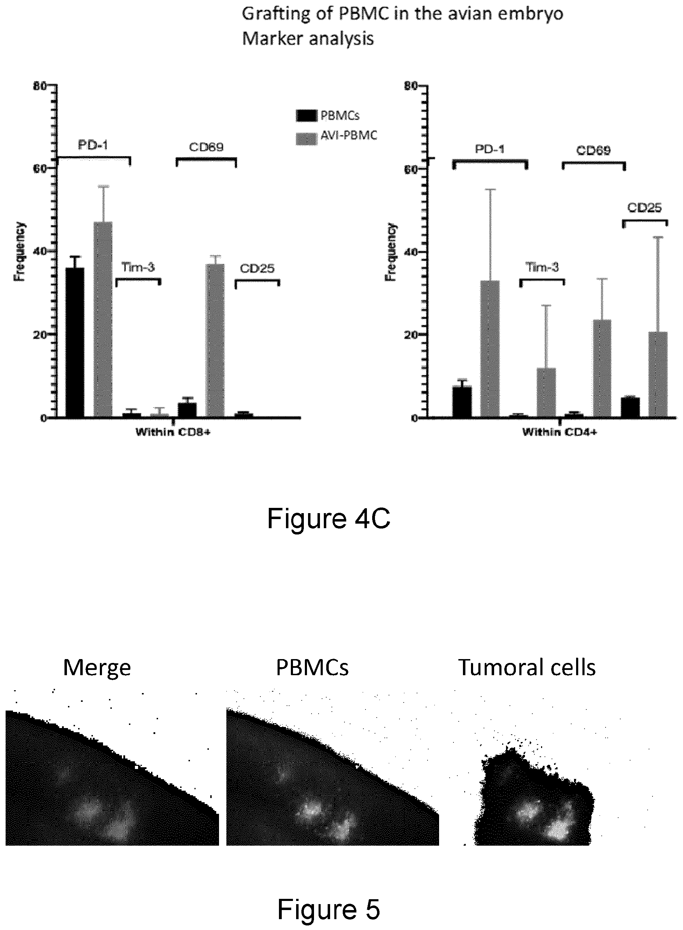

**Figure 5****:** Microphotographs of tumors formed after grating in the avian embryo.
   PBMCs and tumoral cells were co-grafted in a targeted site of the chicken embryo. Prior to the grafting, both pools of cells were labeled with a fluorescent vital tracer (red for PBMCs and green for tumoral cells). Two days post-grafting, the embryos were collected and imaged with a confocal microscope to visualize the grafted cells. A PBMC cellular component was present within the tumors formed.
   Merge) Both types of cells are visualized; PBMCs) PBMCs only are visualized; Tumoral cells) Tumoral cells only are visualized.

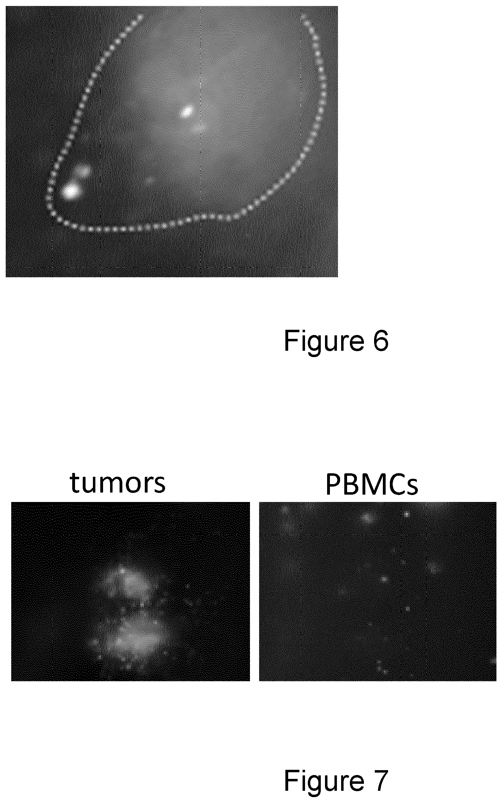

**Figure 6****:** Magnified view of immune cells (white) in human cell tumors formed in the avian embryo (brighter halo). PBMCs were injected in the vasculature, tumoral cells grafted in targets sites of the embryo.
**Figure 7****:** Magnified view of individual tumoral cells in a tumor (tumors) and of immune cells (PBMCs) that were injected in the vasculature of the chick embryo.

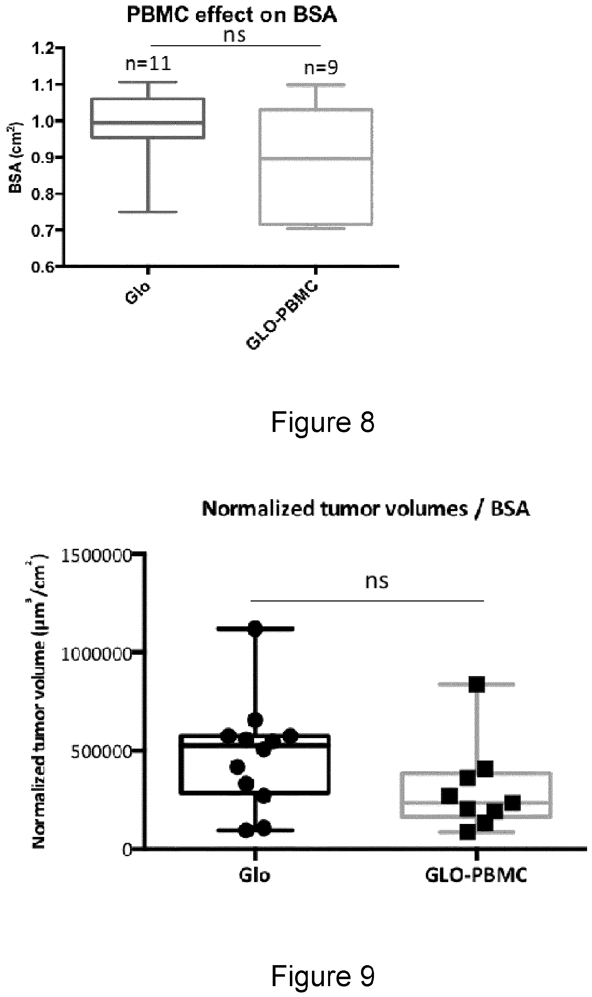

**Figure 8****:** Body/Size Area (BSA), an indicator of the size and the weight of individual embryos, were measured for embryos that were grafted with Pmela cells alone (Glo) and embryos co-grafted with Pmela cells and PBMCs (GLO-PBMC). No statistical difference of BSA was observed.
**Figure 9****:** Measures of tumor volume comparing conditions when Pmela cells were grafted alone or in combination with human PBMCs (according to method 1). The graph shows no statistical difference between both conditions. The data were normalized to the Body Size Area (BSA) of the embryos.
**Figure 10****:**
   (10A) Representation of proportions of immune cell sub-populations after co-graft of PBMCs with two different type of cancer cells (Pmela cells: GLO; MDAM cells: MDAM). Values are expressed in ratios of total identified lymphocytes (CD3+).
   (10B) Expression of surface cellular markers (PD1, TIM-3, CD69 and CD25) is measured by FACS for CD4 + and CD8+ subpopulations of lymphocytes T: on the left CD8+, on the right CD4+ (over alive CD45+CD3+ population)
   Sorted cells are the following: PBMCs alone before grafting (black), AVI-PBMCs after grafting alone in the embryo (dark grey), and AVI-PBMCs +MDAMB231: after co-grafting of tumor cells and PBMCs (light grey). Measures are performed 48 hours post-grafting.

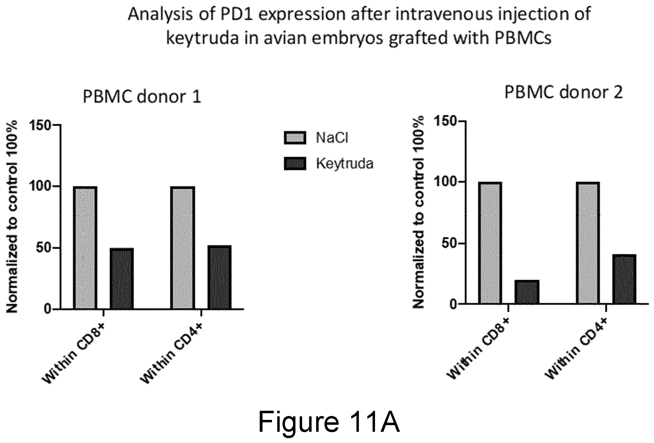

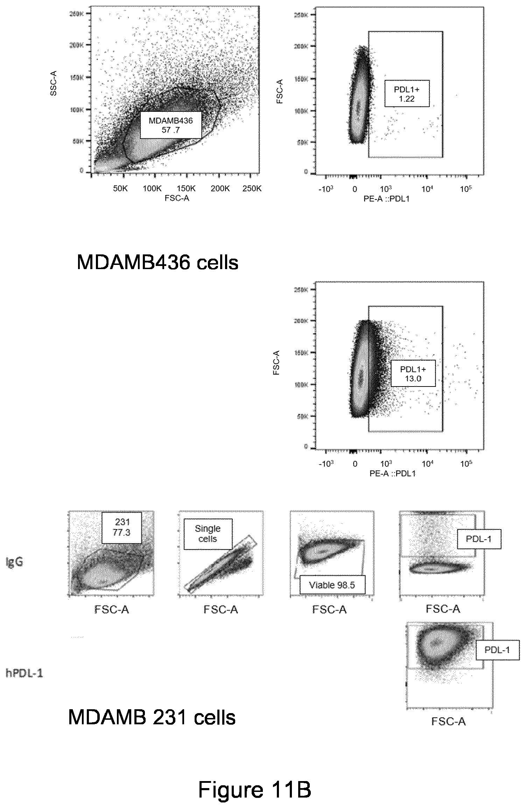

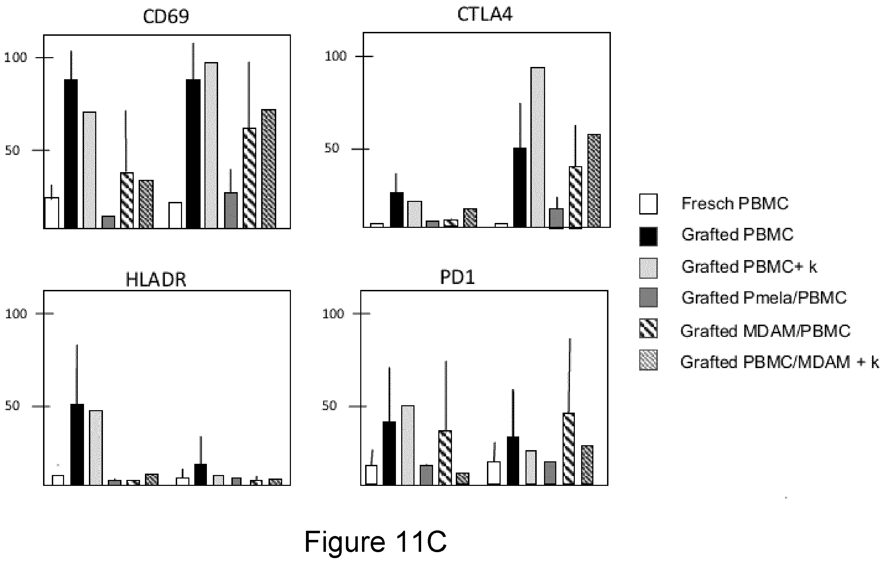

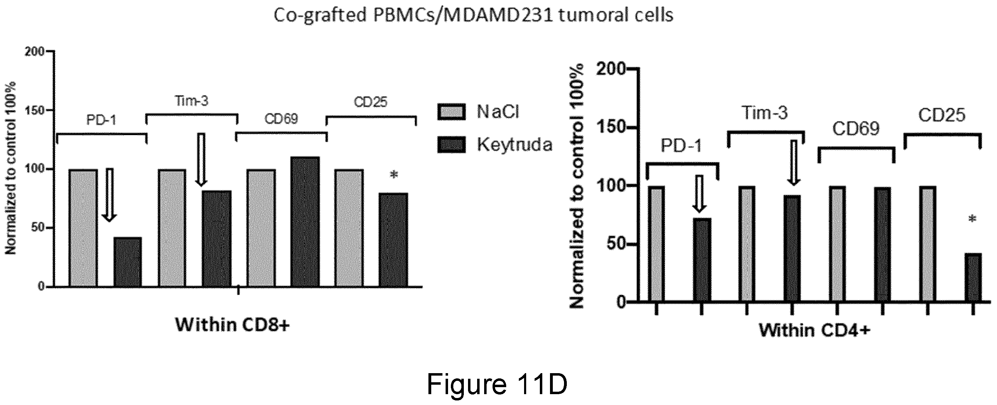

**Figure 11****: Keytruda effects**
   (11A) Subpopulations of cells were identified by FACS after Percoll isolation of PBMCs from collagenase-treated embryos. Detection of PD1 surface expression by FACS. Anti-PD1 (Keytruda) is able to bind to PD1 expressed by human T cells from grafted PBMCs, and thus compete with the anti-PD1 antibody used for detection of PD1 in FACS analysis, leading to reduced PD1 detection. Thus, when Keytruda reaches its target, it results in reduction of detected PD-1 levels.
   Analysis of PD1 expression after intravenous injection of Keytruda (diluted at 5mg/ml), or NaCl for negative control, in avian embryos grafted with PBMCS. Negative controls are arbitrary considered to be 100% of CD4+ and CD8+ cells (within alive CD45+CD3+ population). Quantification of CD8+ and CD4+ cells in Keytruda-treated embryos is expressed as normalized to the controls.
   (11B) PDL1 expression on MDAMB436 breast cancer cells (top figure) and on MDAMB 231 cells (bottom figure) is measured by FACS after grafting into the embryo.
   (11C) PBMC were co-grafted with tumoral cells: Pmela (a PDL1 negative cell line) or MDAMB436 (a PDL1 weak positive cell line). Thereafter, PBMC cells grafted and isolated were treated or not with immunostimulatory anti-human-PD1 Keytruda (K): +K means in presence of Keytruda.
   Markers of activation (CD69, HLADR on the left) and exhaustion (CTLA4, PD1, on the right) were determined in PBMCs after co-grafting with human tumoral cells (Pmela or MDAM) in an avian embryo. Values are expressed in frequency over CD4+ and CD8+ populations in ratios of total identified lymphocytes (CD3+CD4+ and CD3+CD8+). Standard Deviations show data from a pool of PBMC from different healthy donors. Left Graph: CD4+, right graph: CD8+.
   (11D) Repartition of T CD8+ cells (on the left) and CD4+ cells (on the right) after treatment with keytruda for 24 hours (in dark grey) or NaCl (light grey) of embryos co-grafted with PBMCs and MDAMD231 tumoral cells (over alive CD45+CD3+ population).
   Results are presented in % of the control amount; white arrows point the decreased expression of exhaustion markers (PD1 and TIM-3) in the keytruda (5 mg/ml) treatment conditions.

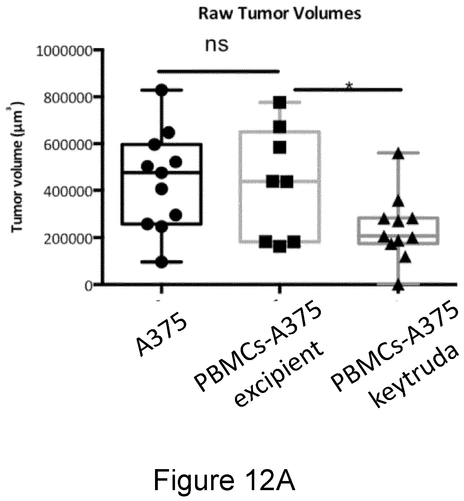

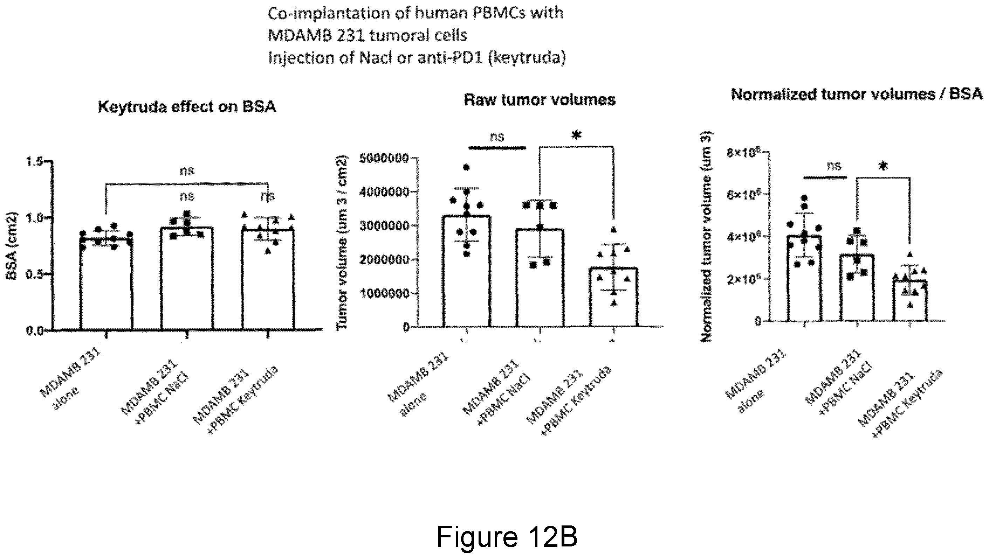

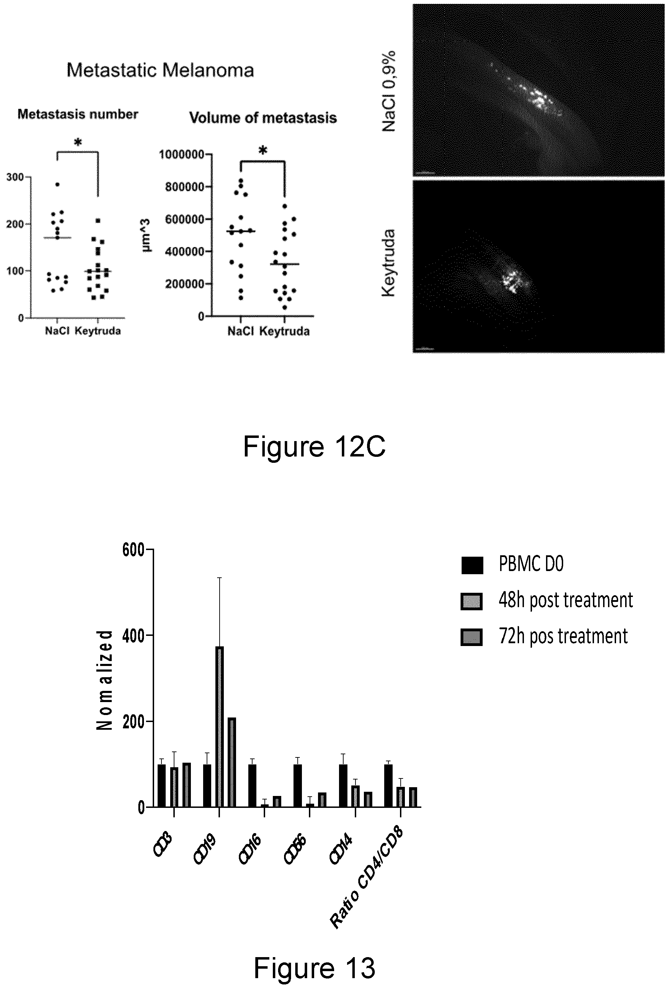

**Figure 12****: Size of tumors in grafted chick embryos**
   (12A) Analysis of the effects of an anti-PD1 treatment (Keytruda) on the volume of tumors formed by co-grafting A375 melanoma cells with human PBMCs, said tumors being formed in the avian embryo model.
   (12B) Effect of anti-PD1 (Keytruda) administration to avian embryos co-grafted with human PBMCs and MDAMB 231 tumoral cells, on the tumor volume
   Left panel: effect of Keytruda on body size area (BSA); Middle panel: Histogram of raw tumoral volumes; Right panel: normalized tumor volumes/ BSA
   All results show a significant reduction of tumor volume in the anti-PD1-treated condition, compared to NaCl-treated embryos.
   (12C) Effect of anti-PD1 (Keytruda) administration to avian embryos co-grafted with human PBMCs and tumor sample from a patient affected by a metastatic melanoma. Metastasis number (on the left) and volume of metastatic tumors (in the middle) have been assessed. On the right, pictures show the extension of the tumor after NaCl treatment (on top) and after Keytruda treatment (on the bottom).
**Figure 13****. Analysis of PBMCs 48 hours and 72 hours post-grafting**
   Monitoring of PBMC subpopulation repartition after grafting in the avian embryo. Embryos were harvested 48h (light grey) or 72h post graft (dark grey). Subpopulations are observed over alive CD45+ population and compared to PBMC before graft (PBMC D0).

### DETAILED DESCRIPTION OF EMBODIMENTS OF THE INVENTION

The present invention concerns a chimeric gallinaceous bird embryo comprising both types of exogenous cells:
a. At least one population of cancer cells, and
b. At least one population of immune cells,

wherein said exogenous cancer cells are present in at least one tissue of said embryo, and said exogenous immune cells are present in at least one tissue of said embryo and/or circulate in the blood vessels of said embryo,
wherein the gallinaceous bird embryo is from a chicken (*Gallus gallus*) or from a quail (*Coturnix japonica*)*,* and
wherein said embryo tissue designates any tissue of the embryo with the exception of the extra-embryonic annexes and the lumen of tubes and vessels.

Unless stated otherwise, the following terms and phrases as used herein are intended to have the following meanings in the sense of the invention.

The term "gallinaceous bird" refers to a bird of the order Galliformes (or gallinaceans) which includes chicks, quails, turkeys, pheasants, peacocks, guinea fowl, and other farmyard animals. In the context of the invention, the embryo will be from a chicken (*Gallus gallus*) or from a quail (*Coturnix japonica*)*,* two species commonly used in the laboratory.

The term "gallinaceous bird embryo" refers to a fertilized gallinaceous bird egg in which an embryo develops normally, under suitable conditions, notably by being placed in an incubator heated to a temperature of 35° C to 42° C.

In a specific embodiment of the invention, the gallinaceous bird embryo is a chicken embryo.

The term "chimeric embryo" designates an embryo possessing cells from said embryo, designated as endogenous cells, and further exogenous cells from at least one other organism, said exogenous cells becoming an integral part of the embryo following their acceptance as graft, and continuing their development within the tissues of the recipient embryo. This chimeric embryo is not intended to develop sufficiently so as to create an adult chimeric organism, but is used only to support exogenous cells during a short period of time. This gallinaceous bird embryo will not produce a chimeric living organism, but will be sacrificed according to the ethical rules in force as soon as the study involving the populations of exogenous cells is completed.

The terms "exogenous cells" designate cells from another organism that have been introduced into a recipient organism, in the present case a gallinaceous bird embryo.

The term "population of cells" designates a group of similar cells, having all the same nature and function. For example, a "population of immune cells" would be lymphocytes T, another population would be lymphocytes B, and another population would be macrophages.

The term "cancer" refers to the pathology characterized by the presence in an organism of malignant cells formed from the transformation, by mutations or genetic instability, of initially normal cells of the organism affected by this pathology.

The terms "cancer cells" designate malignant cells originating from a liquid or a solid tumor. A "population of cancer cells" would be, for example, melanoma cells, another population would be glioblastoma cells, another population would be breast cancer cells.

The terms "immune cells" designate cells of the immune system, all designated as "white blood cells". These cells can be categorized in two major families: neutrophils and Peripheral blood mononuclear cell (PBMC) i.e. blood cells having a round nucleus. While they are both issued from the same hematopoietic progenitor cells, the term "immune cells" do not include erythrocytes also designed as "red blood cells".

Said PBMCs include:
- lymphocytes including T-cells, B-cells and NK (Natural Killer) cells,
- monocytes, macrophages and dendritic cells.

The term "tissue" designates an ensemble of similar cells and their extracellular matrix from the same origin, that carry out a specific function in an organism. A tissue can be an organ, a muscle, a connective tissue, an epithelium, a gland, a nervous tissue (central or peripheral) or an embryonic tissue whose specific function is still unknown. Therefore, an "embryo tissue" designates an embryonic tissue, not yet completely differentiated, representative or homolog to an adult tissue.

Moreover, in the sense of the invention, an "embryo tissue" designates specifically any tissue of the embryo, with the exception of:
- the extra-embryonic annexes of said embryo, such as the chorioallantoic membrane (CAM); and
- the lumen of tubes (such as neural tube) and vessels (such as blood vessels).

The phrase "present in at least one tissue of said embryo" designates the presence, in a tissue of the bird embryo in the sense of the present invention, of exogenous cells. These cells are preferentially aggregated into the form of a tumor.

The phrase "circulate in the blood vessels of said embryo" designates the presence, in the blood circulation of the bird embryo i.e. in the blood vessels of said embryo, of circulating exogenous immune cells.

### The population of immune cells present in the bird embryo

The chimeric gallinaceous bird embryo comprises at least one population of immune cells.

Preferentially, this at least one population of immune cells is chosen among the following: Peripheral Blood Mononuclear Cells (PBMCs), lymphocytes including T-cells, B-cells and NK (Natural Killer) cells.

In a specific embodiment, all populations of immune cells originate from one organism, in particular from the same Human Being. Immune cells from Human Beings can be easily collected by taking of a blood sample followed by a separation of the different type of blood cells.

In a specific embodiment of the invention, the chimeric gallinaceous bird embryo comprises only one population of immune cells.

In a more specific embodiment of the invention, the population of immune cells consists in Peripheral Blood Mononuclear Cells (PBMCs).

In another specific embodiment of the invention, the population of immune cells consists in lymphocytes.

In another specific embodiment of the invention, the population of immune cells consists in lymphocytes B (B-cells).

In another specific embodiment of the invention, the population of immune cells consists in lymphocytes T (T-cells).

In another specific embodiment of the invention, the population of immune cells consists in genetically engineered CAR-T cells.

Genetically engineered CAR-T cells designate CAR-T cells that have been modified genetically, according to any method well known by the man skilled in the art, in order to express specific receptor(s) targeting specific tumoral epitope(s). These cells are able to target and to recognize tumoral cells in order to destroy them. In the sense of the invention, genetically engineered CAR-T cells can be autologous (issued from a patient before their re-injection to said patient) or allogenic (issued from donors and modified in order to be injectable to any person).

Advantageously, these genetically engineered CAR-T cells express chimeric receptors able to recognize tumor specific antigens, to induce T-cell activation and to sustain a co-stimulation signal.

### The population of cancer cells present in the bird embryo

The chimeric gallinaceous bird embryo comprises at least one population of cancer cells.

In a specific embodiment, all populations of cancer cells originate from one organism, in particular from the same Human being. Cancer cells from Human beings can be obtained by any technique known by the man skilled in the art.

In a specific embodiment of the invention, the chimeric gallinaceous bird embryo comprises only one population of cancer cells.

Exogenous cancer cells present in at least one tissue of said embryo may be of any origin. In particular, these cancer cells can be from immortalized cancer cell lines, or from tumor samples.

In a specific embodiment of the invention, exogenous cancer cells are issued from a tumor of a patient.

According to an embodiment of the invention, exogenous cancer cells are selected from the group consisting of: cells derived from primary or secondary brain tumors such as glioblastoma cells or glioma cells, lung cancer cells in particular "EGFR-mutant" lung cancer cells, breast cancer cells in particular HER2+/ER+ mammary tumor cells, prostate cancer cells, sarcoma cells, melanoma cells, germ cell tumor cells, lymphoma cells in particular follicular lymphoma cells, liver cancer cells, digestive cancer cells and ovarian cancer cells.

### Features of both population of cells

The chimeric gallinaceous bird embryo of the invention comprises both types of exogenous cells. In a specific embodiment of the invention, both populations of exogenous cells are human cells.

In another specific embodiment of the invention, both populations of exogenous cells are issued from the same organism, in particular from the same cancer patient.

In another embodiment of the invention, both populations of exogenous cells are issued from at least two different organisms, for example:
- the at least one population of cancer cells is issued from a tumor of a patient, and
- the at least one population of immune cells is a population of allogenic CAR-T cells issued from donors, and genetically engineered.

In this configuration, the chimeric embryo is used for testing the capabilities of the allogenic CAR-T cells to recognize and/or destroy cancer cells from a specific patient.

Another example of this embodiment is the following:
- the at least one population of cancer cells is issued from an immortalized cell line, and
- the at least one population of immune cells is issued from a patient or a healthy donor.

In this configuration, the chimeric embryo is used for testing the capabilities of the immune cells from a patient to recognize and/or destroy specific cancer cells.

Another example of embodiment is the following:
- the at least one population of cancer cells is issued from patient-derived xenograft (PDX) mouse, and
- the at least one population of immune cells is issued from a patient or a healthy donor.

In this configuration, the chimeric embryo is used for testing the capabilities of the immune cells from a patient/healthy donor to recognize and/or destroy specific cancer cells.

In another configuration:
- the at least one population of cancer cells is issued from a patient or a human cell line or a mouse PDX mouse, and
- the at least one population of immune cells is issued from humanized mouse model in which human immune system has been reconstituted.

In this configuration, the chimeric embryo is used for testing the capabilities of the immune cells to recognize and/or destroy specific cancer cells.

In another preferred embodiment of the invention, at least one population of exogenous cells is labelled. Preferably, both populations of immune and cancer cells are labelled, notably with two distinct colors or markers in order to distinct them.

### Localization of exogenous cells in the bird embryo

In the chimeric gallinaceous bird embryo of the invention:
- exogenous cancer cells are present in at least one tissue of said embryo, and
- exogenous immune cells are present in at least one tissue of said embryo and/or circulate in the blood vessels of said embryo.

Accordingly, the present invention relates to three distinct configurations:
(1) a chimeric gallinaceous bird embryo wherein cancer cells and immune cells are present in at least one tissue of said embryo;
(2) a chimeric gallinaceous bird embryo wherein cancer cells are present in at least one tissue, and all immune cells circulate in the blood vessels of said embryo; and
(3) a chimeric gallinaceous bird embryo wherein cancer cells and immune cells are present in at least one tissue, and some immune cells circulate in the blood vessels of said embryo.

In the three configurations, cancer cells are present in at least one tissue of said embryo. Cancer cells may all regroup in only one tissue of said embryo, or be present in two, three, four, five or more different tissues.

In a specific embodiment, cancer cells form at least one tumor, i.e. a mass of cancer cells inserted into the tissue.

In a preferred embodiment of the configurations (1) and (3), both types of exogenous cells are localized in the same at least one embryo tissue, in particular immune cells are present in the tumor(s) formed by cancer cells. More particularly, immune cells have infiltrated the tumor(s). Cancer cells may have formed one tumor or more.

This configuration is particularly advantageous since it reflects the *in vivo* situation, where a tumor is infiltrated with immune cells of the body. This physical association of exogenous cancer and immune cells in the recipient embryo reflects the structure of a tumor in a body and therefore, the chimeric gallinaceous bird embryo of the invention is a relevant animal model for studying interactions between tumor cells and immune cells.

In a preferred embodiment of the invention, both types of exogenous cells are physically associated. In particular, immune cells and cancer cells form a solid tumor infiltrated with immune cells, in at least one tissue of the embryo.

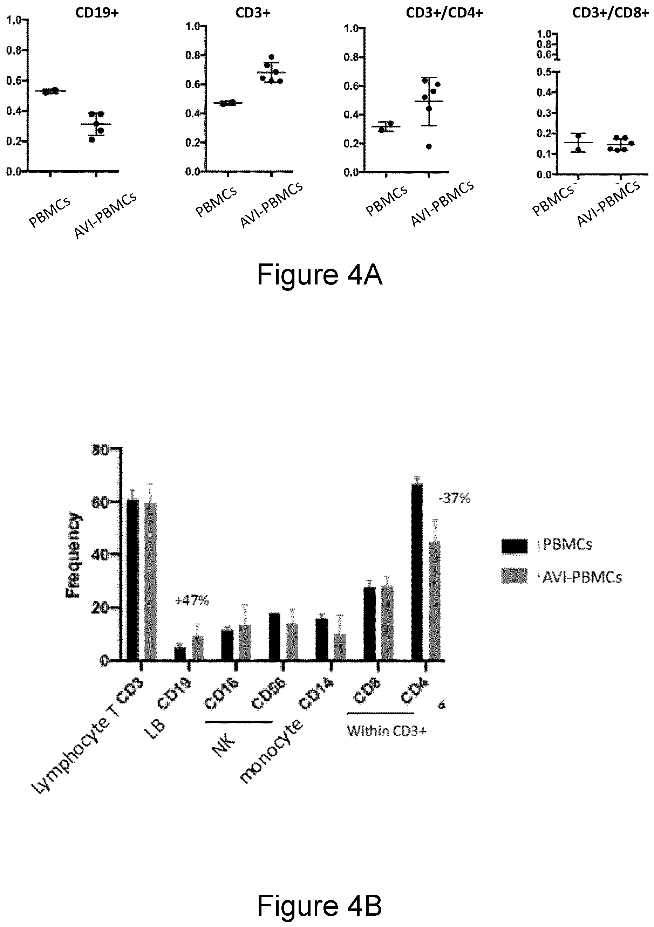

Advantageously, the tumor is infiltrated with different subpopulations of immune cells, in particular with CD4+ and CD8+ T cells; examples show the diversity of the immune cells populations that are present in the tumor formed in the grafted embryo. As is shown in figure 4B, all subpopulations of grafted PBMCs are maintained in the embryo after grafting.

Advantageously, the tumor is infiltrated with immune cells that express markers of activation and/or exhaustion. In particular, said immune cells express PD1 and/or TIM-3 exhaustion markers. Interestingly, immune cells expressing PD1 are responsive to a treatment with an anti-PD1 compound.

In a specific embodiment of the invention, the formed tumor in the embryo comprises exogenous immune cells that express markers of activation (CD69, CD25) and/or exhaustion (PD1, TIM-3).

Advantageously, in the tumor formed in the gallinaceous bird embryo after grafting, it exists a "dialog" between immune and tumor cells that is representative of the physiology of a tumor in a patient body.

In a preferred embodiment of the three configurations, exogenous cancer cells are present in at least one embryo tissue which is relevant with the nature of the population of cancer cells, in particular is representative - i.e., the equivalent tissue or organ - of tissues/organs in which primary or secondary tumor(s) form(s) in cancer patients.

A "representative tissue" or "equivalent tissue", both terms being used interchangeably, designates a gallinaceous bird embryo tissue that is equivalent to a non-embryonic mammalian tissue, but in its embryonic state and in the configuration of a gallinaceous bird organism. It designates a tissue that mimics the tissular, cellular and/or molecular aspects of a specific non-embryonic mammalian tissue.

For example, embryonic colon issued from endoderm is representative of the colon organ of a mammal; when exogenous cancer cells are cancer colon cells, they are present in said endodermic germ layer.

Another example is the case wherein exogenous cancer cells are breast cancer cells, which tend to metastasis in bone tissues; exogenous cancer cells may, in this case, be present in embryonic bones, issued of the mesoderm germ layer.

### Processes of obtention of chimeric gallinaceous bird embryos of the invention

The present invention also concerns a process for obtaining a chimeric gallinaceous bird embryo such as described previously, i.e. comprising both types of exogenous cells:
a. At least one population of cancer cells, and
b. At least one population of immune cells,

said process comprising the following successive steps:
   - Introducing both populations of exogenous immune cells and cancer cells into a gallinaceous bird embryo, wherein the gallinaceous bird embryo is at a developmental stage between stage HH10 and stage HH30, and
   - incubating the chimeric embryo for at least 6 hours, preferably at a temperature comprised between 35° C and 42° C,
wherein said exogenous cancer cells are introduced into at least one tissue of said embryo, and said exogenous immune cells are introduced in at least one tissue of said embryo and/or in the blood vessels of said embryo,
wherein the gallinaceous bird embryo is from a chicken (*Gallus gallus*) or from a quail (*Coturnix japonica*)*,* and
wherein said embryo tissue designates any tissue of the embryo with the exception of the extra-embryonic annexes and the lumen of tubes and vessels.

The term "introduction" designates any technique useful for creating a chimeric embryo, comprising exogenous cells further its own cells. In particular, the introduction of cells is a graft of exogenous cells into a specific tissue of the embryo.

The step of incubation shall be performed according to standard techniques, in particular in a humidity-saturated incubator.

The temperature of incubation will be adapted to the nature of the gallinaceous bird embryo according to the knowledge of the man skilled in the art. In a specific embodiment, the temperature of the incubator is comprised between 35°C and 42°C, with the range bounding values being included. Preferentially, the incubation temperature is comprised between 37°C to 40° C, and is preferably of about 38.5°C for a chicken embryo.

The incubation time is of at least 6 hours, time that is necessary for obtaining first aggregation of cancer cells into at least one tissue of the embryo. Preferentially, the incubation period is of at least 8 hours; at least 10 hours; at least 12 hours; at least 16 hours; at least 24 hours; at least 36 hours; or at least 48 hours. Incubation periods are generally of about 24 hours or of about 48 hours.

Preferentially, incubation and analysis of the embryo is stopped before complete embryonic development of the embryo (i.e., 21 days post fertilization for a chicken embryo), and more preferentially, the process of incubation and analysis is finalized in the first two-thirds of the period of embryonic development, i.e., before the fourteen day post fertilization.

Both types of process are objects of the present invention and are detailed below.

In a first implementation, the process of the invention comprises the following successive steps:
- co-grafting both populations of immune cells and cancer cells within at least one tissue of a gallinaceous bird embryo at a developmental stage between stage HH10 and stage HH30, and
- incubating the chimeric embryo for at least 6 hours at a temperature comprised between 35°C and 42°C.

The term "co-grafting", in the sense of the invention, refers to the introduction of both types of exogenous cells into at least one tissue of the recipient embryo, during one and only procedure.

Exogenous cells can be grafted in the form of:
- suspended cells, injected into the target tissues;
- a solid piece (block) of tumor tissue comprising both immune and cancer cells;
- an aggregate/homogenate of isolated cells.

The grafting of exogenous cells into the recipient gallinaceous bird embryo is performed according to the methods well known to persons skilled in the art. The gallinaceous bird embryo is indeed easily accessible, after having made a small opening in the eggshell. In particular, grafting of the cancer cells is performed using a pneumatic microinjector (Picopump PV830, World Precision Instruments). The technique is in particular presented in patent applications WO 2016/05398 and WO 2017/103025.

Several developmental stages of the gallinaceous bird embryo have been previously defined as a function of post-fertilization incubation time and determined according to the criteria defined by Hamburger and Hamilton (1951, J Morphol.).

According to the invention, at the time of the grafting of exogenous cells, the gallinaceous bird embryo is at a developmental stage between stage HH10 and stage HH30.

Preferably, at the time of the introduction of exogenous cells, the gallinaceous bird embryo is at a developmental stage between stage HH12 and stage HH25. This developmental phase of the embryo, taking place between 40 hours and 4.5 days post-fertilization, is characterized by many key events of embryogenesis, among which the appearance of somites, the subdivision of the large brain regions, the curvatures of the various regions of the embryo, and the formation of numerous organs.

According to another preferred aspect, at the time of the introduction of exogenous cells, the gallinaceous bird embryo is at a developmental stage between stage HH10 and stage HH18, between stage HH10 and stage HH15, or between stage HH12 and stage HH16.

Preferably, the grafting of cancer cells is performed on a recipient gallinaceous bird embryo at a developmental stage between stages HH13 and HH15, *i.e.,* between 48 and 55 hours post-fertilization, and preferably between 50 and 53 hours post-fertilization (stage HH14).

In a second implementation, the process of the invention comprises the following steps:
- Grafting the at least one population of cancer cells within at least one tissue of a gallinaceous bird embryo at a developmental stage between stage HH10 and stage HH30,
- Injecting said at least one population of immune cells into the blood vessels of said embryo, and
- incubating the grafted embryo for at least 6 hours at a temperature comprised between 35°C and 42°C.

The grafting step and incubation steps have been previously described, and are performed as previously indicated.

In this implementation of the process, the step of "injecting cells" corresponds to a step where immune cells are introduced into the blood circulation of the embryo by any technique known by the man skilled in the art.

This step of injection may be performed concomitantly to the step of grafting of cancer cells, or before or after. In particular, the step of injection of immune cells can be performed at the later stage of the development of the embryo, i.e. after the HH30 stage. However, in a preferred embodiment, both types of exogenous cells are introduced into the embryo at the same time.

In some cases, as shown in the examples section, immune cells will join the cancer cells implanted in at least one tissue and infiltrate the tumor(s). In some other cases, immune cells will circulate into the blood vessels of the embryo and their ability to act on the development of cancer cells from the blood circulation will be tested. In some other cases, immune cells will both join the cancer cells and circulate into the blood vessels of the embryo.

The grafting of the population of cancer cells may be performed in at least one embryo tissue that is relevant with the nature of said population of cancer cells, in particular is representative of tissues/organs in which primary and/or secondary tumor(s) form(s) in cancer patients.

In the sense of the invention, said "relevant" tissue is either in accordance with the origin of cancer cells, or is a tissue which is usually colonized by said cancer cells, i.e. where secondary tumors (metastases) implant.

Exogenous cancer cells may be grafted into the recipient embryo in different tissues, and for example: in the tissue constituting the neural tube (not in the lumen), into the brain tissues, in embryonic tissues prefiguring digestive structures such as the liver, the intestine, or the pancreas, in embryonic tissues prefiguring the skin, the bones and the bone marrow, or in the somitic domain.

### Animal model and uses thereof

The present invention also relates to an animal model for the study of human cancers consisting in a chimeric gallinaceous bird embryo as presented above, or susceptible to be obtained by any one of the processes as described above.

The present invention also relates to an animal model for the study of human cancers obtained by any one of the processes as described above.

This animal model of the invention has numerous uses.

In particular, this animal model can be used for monitoring a patient affected with a cancer. Indeed, such animal model makes it possible to monitor, *ex vivo,* the development of a patient tumor, and in particular after starting a treatment at a time T₀ (e.g., before the beginning of said treatment) and at a time T₁, T₂, T₃ (e.g., after the beginning of the cancer-patient's treatment).

The animal model may also be used for screening therapeutic molecules intended for the treatment of cancer.

In particular, the present invention relates to the use of an animal model of the invention for screening at least one anti-cancer therapy chosen among the following group:
- immunotherapy: therapeutic strategy consisting in promoting the antitumoral activity of the immune cells of the patient,
- chemotherapy: drugs affecting the viability, the metabolism, the motility, and/or the proliferation of cancer cells,
- targeted therapy: use of compound(s) such as antibodies targeting a specific molecule expressed by cancer cells,
- hormonotherapy: use of compound(s) targeting the hormonal molecular signaling,
- anti-cancer drugs, and
- therapeutic enzymes.

More particularly, the animal model of the invention may be used for assessing the activity of genetically engineered CAR-T cells. In a specific embodiment, these CAR-T cells are allogenic CAR-T cells, intended to be used in any patient. Efficacity and safety of these allogenic cells may be tested in an animal model according to the invention.

### EXAMPLES

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

### Materials and Methods

Fertilized *gallus gallus* eggs were obtained from dedicated farms such as EARL MORIZEAU, DANGERS, FRANCE, and maintained at 14° C until use. For running experiments, eggs were incubated at 38,5° C for 52 hours, in order to obtain embryos at development stage HH13 to HH16, according to the Hamilton nomenclature.

For manipulations, a window was opened in the shell.

Human PBMCs and tumoral cells were grafted using a micro-injector in which they are inserted (Pipopump PV830, World Precision Instruments).
(1) In some experiments, human PBMCs were injected into the vessels of the embryo, through the vessels present on the chorioallantoid membrane. Cancer cells are grafted according to the process described in the patent applications WO 2016/05398 and WO 2017/103025.
(2) In other experiments, both types of cells were co-grafted at chosen sites of the embryo, within tissue in which human tumoral cells form tumors, to establish tumors with a stroma of human immune cells.

Embryos were kept in incubation over 24 hours to 48 hours after grafting of cancer cells. They were collected from the eggs and processed for different experiments. In some experiments, the embryos were digested with collagenase, and human cells were isolated for further analysis. In other experiments, embryos were fixed with 4% paraformaldehyde overnight, at 4° C. Embryos were cleared for imaging with light sheet microscope (LaVision Biotec).

### I. Demonstration of the feasibility to graft PBMC in an avian embryo model

### Example 1

Conditions (1): The shell of embryonated chick eggs was opened. Human PBMCs from healthy donners were injected in the vasculature (blood circulation) of the embryo.

Conditions (2): the PBMCs were prepared, labeled with a vital fluorescent tracer, and grafted by injection using a microcapillary in targeted tissues of the avian embryo.

Two days after PBMC injection (1) or grafting (2), embryos were collected, fixed in paraformaldehyde, imaged with a stereomicroscope, and then cleared and imaged using light sheet microscopy.

In other experiments, embryos were digested with collagenase, and PBMCs were purified on Percoll gradient.

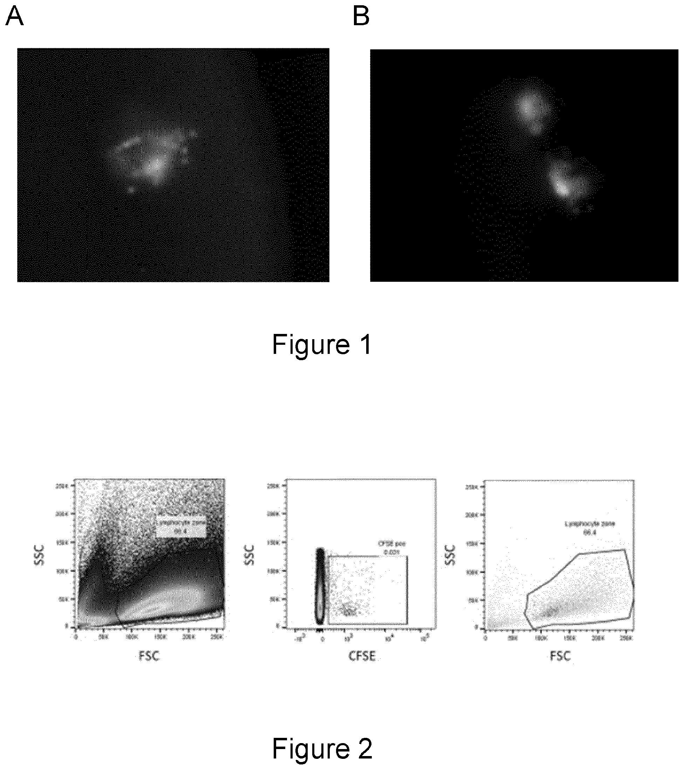

In both experimental conditions, alive PBMCs were found in the avian embryonic tissues, as is shown in figure 1A and 1B.

### Example 2

In another experiment, PBMCs from healthy donors were labeled with a vital fluorescent tracer (CFSE: Carboxyfluorescein succinimidyl ester), and then injected in the vasculature of chicken embryos at 56h post gestation (HH13-HH15).

Two days later, the embryos were collected, digested with collagenase after head removal. PBMCs were isolated using FICOLL and PERCOLL gradient techniques. and fluorescent PBMCs cells were analyzed by FACS.

Results are presented in figure 2, and demonstrate that injected PBMCs are alive 48 hours after introduction into the embryo.

### Example 3

PBMCs were injected in the vasculature of the embryo at different doses. Two days (48 hours) post-injection, isolated PBMCs were counted to determine the percentage of survival.

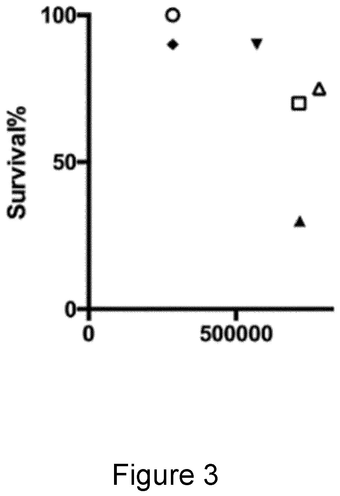

Results are presented in figure 3. Cell survival was compromised at high dose such as 6×10⁵ cells/µl (black triangle), compared to injection of a lower number PBMC (dilution by 2), for which a good survival was observed. It appears that, rather than the absolute number of cells, the concentration of injected cells is an important parameter for cell survival.

### Example 4

PBMC from human blood samples were prepared, and grafted in the tissues of the avian embryo at 56 hours post laying (HH14).

Two days after the graft, embryos were collected and digested using collagenase. Immune cells were purified and the sub-populations of immune cells was analyzed in FACS with molecular markers, in order to determine the proportion of the lymphocyte B (CD19+) and T (CD3+CD4+ and CD3+CD8+) population over the total immune cell population. All main PBMC subpopulations were then monitored: Lymphocyte (B and T), Natural Killer, myeloid cells (here named monocyte) over the total PBMC population (CD45+ cells).

Results are presented in figures 4A, 4B and 4C. "PBMCs" designate immune cells prior to the grafting; "AVI-PBMCs" designate PBMC cells grafted and analyzed after Percoll isolation from collagenase-treated embryos.

In Figure 4A, values are expressed in ratios of total identified lymphocytes (CD19+ and CD3+).

Under these conditions, the proportion of the lymphocyte B population over the total immune cell population was weaker in the chick embryo, compared to the population of fresh PBMCs. This difference of proportions is also observed in murine models of xenograft (PDX).

Figure 4B shows the subpopulation repartition of immune cells, before their grafting (PBMCs) and 48 hours after their grafting in the chicken embryo (AVI-PBMCs). Interestingly, all PBMC subpopulations have been maintained after grafting into the chick embryo.

Figure 4C shows the expression of markers of activation (CD69, CD25) and exhaustion (PD1, TIM-3) by immune cells, before their grafting (PBMCs) and 48 hours after their grafting in the chicken embryo (AVI-PBMCs). Two populations of T lymphocytes are analyzed: CD8+ and CD4+.

Except for CD69 that is significantly increased after grafting in both CD4° and CD8+ lymphocyte T cells, the expression of other markers is maintained or slightly increased after the grafting. The activation of PBMC is rather weak, showing that allogeneic reaction is weak. PBMC grafting does not disturb the development of the embryo, indicating that the host and grafted PBMC can cohabit.

### II. Demonstration of the feasibility to co-graft PBMC in an avian embryo model with a tumor cell line

### Example 5. Introduction of both exogenous populations of immune cells and cancer cells

Human PBMCs were labeled with a vital fluorescent tracer.

Two fluorescent human tumor cell lines were used:
- a primary melanoma cell line derived from a patient sample (Pmela, metastatic BRAF V600 melanoma), also designated as GLO, and
- MDAMB436 cells (triple negative breast cancer cell line, commercially available), also designated as MDAM.

Both type of cells is introduced into the embryo as presented in the Material and Methods section.

Conditions (1): PBMCs were co-grafted with tumor cells at chosen tissues of the embryo.

Conditions (2): PBMCs were injected in the vasculature. Tumor cells were grafted at chosen tissues of the embryo.

Two days after the graft, embryos were imaged with stereomicroscope. Collected embryos were measured and their weight established. Embryos were cleared and images with confocal light sheet microscopy. Images were processed with a software (IMARIS) for quantification of the tumor volume.

With both conditions, the presence of immune cells within the tumors was observed. The immune "contingent" was more robust in terms of cell number with the co-grafting method. Results for the conditions (1) are presented in figure 5. The "Merge" picture represents both types of populations.

Results for the conditions (2) are presented in figures 6 and 7.

In figure 6, a brighter halo (marked with a dotted line) shows immune cells that regroup around the tumors formed in the avian embryo.

In figure 7, individual tumoral cells in a tumor implanted in chicken embryo are labelled (picture on the left), and immune cell from PBMCs are labelled (picture on the right): immune cells regroup around the tumors.

### Example 6. Measure of the BSA of the grafted chicken embryos and tumors volume, with or without immune cells

The indicator Body Size Area (BSA) is measured in order to determine the toxicity of the introduction of PBMCs into the chicken embryos.

Results presented in figure 8 show that no statistical difference of BSA was observed for embryos that were grafted with Pmela cells alone (Glo), or co-grafted with PBMCs (GLO-PBMC).

Results presented in figure 9 represent measures of tumor volume when Pmela cells (Glo) were grafted alone or in combination with human PBMCs (GLO-PBMC), according to conditions (1).

The graph shows no statistical difference between embryos with grafted cancer cells and those with a co-graft of cancer and immune cells. The data were normalized to the Body Size Area (BSA) of the embryos.

### Example 7. Co-grafting of tumor cells and PBMCs

In a chicken embryo, PBMCs were co-grafted with at least one of the two following tumor cell lines:
- Pmela - primary cells from metastatic BRAF V600 melanoma (PBMCs-GLO)
- MDAMB436 and MDAMB231- immortalized cell line from triple negative breast cancer (PBMCs-MDAM)

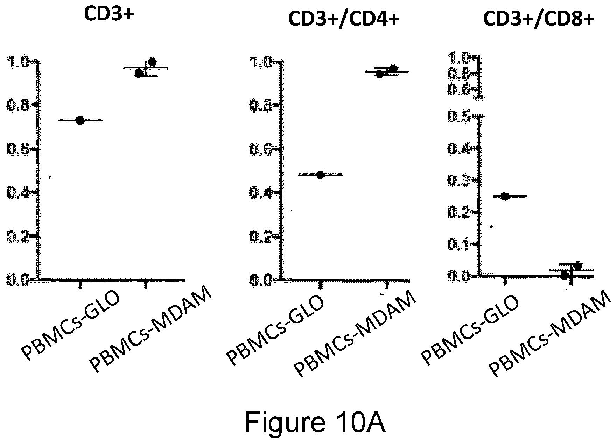

Two days after the graft, PBMCs were purified from the embryos and the molecular markers of the different cell populations were analyzed by cytofluorimetry: CD3+, CD4+ and CD8+. Results are presented in figure 10A.

As is shown, PBMCs react when grafted in the avian embryonic tissues, compared with their resting state prior to the grafting. The presence of tumoral cells inhibits the activity of T cells, resulting in downregulation of CD69 and HLADR markers, bringing their levels back to those of fresh PBMCs. Greater levels of CTLA4 on CD4 and CD8 T cells are observed after grafting of PBMCs. Keytruda administration leads to a minimization of PD1 expression/accessibility on CD8 T cells. PD1 blocking leads to very high levels of CTLA4 on the CD8 compartment, suggesting a compensatory expression.

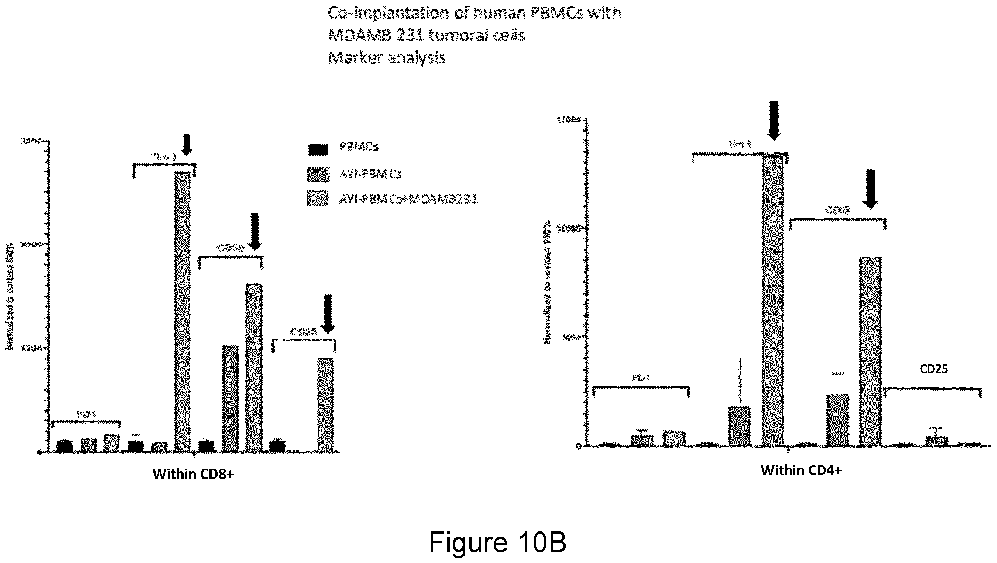

Figure 10B illustrates the pattern of markers expressions on surface of immune cells (CD8+ on the left, CD4+ on the right) after the co-graft of PBMCs and MDAMB231 cells - that express high PDL1 levels - in a chick embryo.

In presence of cancer cells, immune cells included in a tumor present:
∘ an increase in activation markers CD69 and CD25 expression, in particular among CD8+ pool;
∘ an increase of expression of exhaustion markers PD1 and TIM-3 (also designated as immune checkpoint control modulators).

These results illustrate a two-steps reaction of immune cells to the presence of co-grafted tumoral cells:
(i) MDAMB 231 tumoral cells activate T cells, as depicted by increase in CD69 and CD25 expression, compared to PBMCs grafted alone;
(ii) inhibition of the immune response is already visible, as depicted by increase in expression of immune checkpoint control modulators PD1 and TIM-3. This expression pattern reflects the negative immune regulation on lymphocytes by the cancer cells (correlated with the tumor cells "escaping").

Thus, xenografting of human immune cells into the avian embryo induces their activation and their "neutralization". This indicates that T cells are still functional when introduced in the avian embryo, and are able to recognize allogeneic cells.

### III. Demonstration of the feasibility to treat grafted PBMC with immunotherapy

### Example 8. Keytruda (anti-PD1) treatments

This experiment was performed to study the human T cell state of PBMCs introduced into the avian embryo. The T cell compartment is the target of several currently approved checkpoint inhibitors such as αCTLA4 and PD1 axis blockade.

First, expression of PD1 receptors on grafted PBMCs (figure 11A) and expression of PDL1 by cancer cells (figure 11B) were verified. MDAMB436 cells were cultured *in vitro* and immunolabeled with anti-PD L1 antibody to determine the expression of the PD1 ligand, PDL1. Similar experiments were done on Pmela cells and MDAMB231 cells.

Then, MDAMB436 cells or Pmela cells were co-grafted with human PBMC cells.

An anti-PD1 antibody, Keytruda (Pemzolibrumab, Merk), was injected 24 hours post-grafting.At two days (48 hours) post-grafting, PBMCs were isolated from grafted embryos and analyzed by FACS.

Finally, grafted PBMCs were isolated by Percoll density separation and stained cells for markers of activation (CD69/HLADR) and exhaustion (CTLA4/PD1).

### Results are commented below.

### a) PD1 expression by grafted immune cells

Figure 11A illustrates the level of PD1 expression on surface of the cells, within CD8+ and CD4+ T cells from two different donors. After treatment with Keytruda (diluted by 5 fold) which antagonizes the PD1 receptors, detection of PD1 is significantly decreased. These results show that Keytruda does bind to the PD1 receptors of grafted immune cells.

### b) PDL1 expression by grafted cancer cells

Results are presented in figure 11B. Approximately 10% of MDAMB436 cultured *in vitro* present an expression of PDL1 ligand. No PDL1 expression was detected in Pmela cells. PDL1 expression is high in MDAMB231.

### c) T cell state

Among tumor cells, Pmela is a PDL1 negative cell line, and MDAMB436 is a weak PDL1 positive cell line. The grafted avian embryos were treated or not with immunostherapy anti-human-PD1 Keytruda (K).

Subpopulations of cells were identified by FACS after Percoll isolation of PBMCs.

Results are presented in figure 11C. Markers of activation (CD69 and HLADR, on the left) and exhaustion (CTLA-4 and PD1, on the right) were determined for each population of cells:
- Fresh PBMC (not introduced into any embryo)
- Grafted PBMCs
- Grafted PBMCs +K: the embryo has been treated 24 hours with Keytruda, an anti-PD1 antibody
- Grafted Pmela/PBMC: co-graft of immune cells and cancer cells Pmela that do not express PDL1
- Grafted MDAM/PBMC: co-graft of immune cells and cancer cells MDAM, expressing PDL1
- Grafted MDAM/PBMC +K: the embryo has been treated 24 hours with Keytruda, an anti-PD1 antibody.

Standard Deviations show data from a pool of PBMC from different healthy donors. Values are expressed in ratios of the specific marker over CD3+CD4+ and CD3+CD8+ populations.

As a general rule of thumb, CD4 and CD8 T cells isolated post- grafting were showing signs of activation: CD4 and CD8 T cells were largely positive for CD69, whereas CD4 T cells gained HLADR expression.

Regarding inhibitory receptors, we observed greater levels of CTLA4 on CD4 and CD8 T cells after grafting of PBMC whereas greater levels of PD1 were true only for CD4 T cells.

Thus, xenografting of human immune cells into the avian embryo induces their activation. Human immune cell activation is also reported in mice model. This indicate that T cells are still functional when introduced in the avian embryo, and are able to recognize allogeneic cells.

PD1 blocking led to very high levels of CTLA4 on the CD8 compartment, suggesting a compensatory expression.

d) Another experiment was performed with MDAMD231 cells, co-grafted with PBMCs, followed by treatment of the grafted embryo with Keytruda (5 mg/ml).

Expression of activation and exhaustion markers on co-grafted immune cells was quantified. Results are shown in figure 11D.

Expression of exhaustion markers PD1 and TIM-3 is decreased with the Keytruda treatment, which confirms that Keytruda is able to restore the responsiveness of exhausted T lymphocytes.

Activation markers will probably be re-instaurated later during Keytruda treatment (it is probable that 24 hours of administration is not sufficient to re-instaure expression of these activation markers). As well higher concentration of Keytruda should lead to increased cell activation.

In conclusion, 24 hours of Keytruda administration is able to initiate a reactivation process of immune cells, as depicted by the decrease of PD1 and TIM-3 expression.

### IV. Demonstration of the potential of the grafted avian embryo of the invention, as model for monitoring response to immunotherapy treatment

### Example 9. PBMC co-grafted with A375 cell line

To determine whether tumors composed of mixed human tumoral cells and immune cells are sensitive to anti-PD1 antibody treatment, the human melanoma A375 cell line was selected for the experiments. Indeed, this cell line was recently reported to express significant levels of PDL1 and to respond to anti-PD1 immunotherapy (Kuryk L, Møller AW, Jaderberg M, 2019).

A375 melanoma cells were cultured and co-grafted with human PBMC cells as previously described.

An anti-PD1 antibody, Keytruda, or a negative control (excipient) was injected into the embryo 24 hours post-grafting.

Two to three days post-grafting, embryos were collected and processed for imaging with a confocal light sheet microscope. The volume of the tumors was quantified using IMARIS software.

Results are presented in figure 12A: in presence of Keytruda, the volume of the tumor decreases significantly, which demonstrates that the immune cells present around the cancer cells respond positively to this immunotherapeutic treatment.

### Example 10. PBMC co-grafted with MDAMB 231 cells or patient cells

The same experiment of example 9 was performed with other cancer cells:
- MDAMB 231, well-known to be responsive to immunotherapy, and
- a tumor sample issued from a patient affected by a metastatic melanoma.

Figure 12B shows the effect of Keytruda administration to avian embryos co-grafted with human PBMCs and MDAMB 231 tumoral cells, on the tumor volume (see middle and right panels).

A significant reduction of tumor volume is observed in the anti-PD1-treated condition, compared to Nacl administration.

Figure 12C shows the effect of Keytruda administration to avian embryos co-grafted with human PBMCs and a tumor sample from a patient affected by a metastatic melanoma.

A significant reduction of the metastatic-volume is observed in Keytruda-administration conditions.

Also, the number of metastases formed by patient tumoral cells in the avian embryonic tissues has been assessed by the following of fluorescently-labelled tumor cells, and subsequent quantification of the fluorescent signal in the embryo.

After administration of Keytruda, the ability of tumor cells to metastasis is significantly decreased as is shown on the histogram entitled "metastasis number".

Both histograms show that anti-PD1 administration results in significant reduction of the volume and number of metastases formed by patient tumoral cells in the avian embryonic tissues.

### Example 11. Analysis of PBMCs 48 hours and 72 hours post-grafting.

Embryos were harvested 48h (light grey) or 72h post graft (dark grey). Subpopulations are observed over alive CD45+ population and compared to PBMC before graft (PBMC D0). Results are presented in figure 13.

All main PBMC subpopulations are found alive. Some populations are also in higher proportion at 72 hours, compared to 48h post grafting (Natural Killer population). Allogeneic reaction of PBMC against avian host does not impact the embryo development after 72h. Therefore, 48 hours of immunotherapy treatment can be achieved with the model.

### REFERENCES

### PATENTS

WO 2015/074050
US 2013/0171680
WO 2016/05398
WO 2017/103025
WO 2020/075168

### BIBLIOGRAPHIC REFERENCES

Park TS, Zambidis ET, Lucitti JL, Logar A, Keller BB, Péault B. Human embryonic stem cell-derived hematoendothelial progenitors engraft chicken embryos. Exp Hematol. 2009 Jan;37(1):31-41.
Carter R, Mullassery D, See V, Theocharatos S, Pizer B, Losty PD, Jesudason E, Moss DJ. Exploitation of chick embryo environments to reprogram MYCN-amplified neuroblastoma cells to a benign phenotype, lacking detectable MYCN expression. Oncogenesis. 2012 Aug 27;1:e24.
Boulland JL, Halasi G, Kasumacic N, Glover JC. Xenotransplantation of human stem cells into the chicken embryo. J Vis Exp. 2010 Jul 11;(41):2071.
Busch C, Krochmann J, Drews U. Human melanoma cells in the rhombencephalon of the chick embryo: a novel model for brain metastasis. Exp Dermatol. 2012 Dec;21(12):944-7.
Jayachandran A, McKeown SJ, Woods BL, Prithviraj P, Cebon J. Embryonic Chicken Transplantation is a Promising Model for Studying the Invasive Behavior of Melanoma Cells. Front Oncol. 2015 Feb 16;5:36.
Joel M, Sandberg CJ, Boulland JL, Vik-Mo EO, Langmoen IA, Glover JC. Inhibition of tumor formation and redirected differentiation of glioblastoma cells in a xenotypic embryonic environment. Dev Dyn. 2013 Sep;242(9):1078-93.
Leach, D. R., Krummel, M. F. & Allison, J. P. Enhancement of Antitumor Immunity by CTLA-4 Blockade. Science 271, 1734-1736 (1996).
McLane, L. M., Abdel-Hakeem, M. S. & Wherry, E. J. CD8 T Cell Exhaustion During Chronic Viral Infection and Cancer. Annu. Rev. Immunol. 37, 457-495 (2019).
Feins, S., Kong, W., Williams, E. F., Milone, M. C. & Fraietta, J. A. An introduction to chimeric antigen receptor (CAR) T-cell immunotherapy for human cancer. Am. J. Hematol. 94, S3-S9 (2019).
Martinez, M. & Moon, E. K. CAR-T Cells for Solid Tumors: New Strategies for Finding, Infiltrating, and Surviving in the Tumor Microenvironment. Front. Immunol. 10, 128 (2019)
Jung, J., Seol, H. S. & Chang, S. The Generation and Application of Patient-Derived Xenograft Model for Cancer Research. Cancer Res. Treat. Off. J. Korean Cancer Assoc. 50, 1-10 (2018).
Siegler EL, Wang P. Preclinical Models in Chimeric Antigen Receptor-Engineered T-Cell Therapy. Hum Gene Ther. 2018 May;29(5):534-546. Hamburger V, Hamilton Hl. A series of normal stages in the development of the chick embryo. J Morphol. 1951 Jan;88(1):49-92. PubMed PMID: 24539719.
Kuryk L, Møller AW, Jaderberg M. Combination of immunogenic oncolytic adenovirus ONCOS-102 with anti-PD1 pembrolizumab exhibits synergistic antitumor effect in humanized A2058 melanoma huNOG mouse model. Oncoimmunology. 2018 Oct 29;8(2):e1532763.

## Claims

1. Chimeric gallinaceous bird embryo comprising both types of exogenous cells:
a. At least one population of cancer cells, and
b. At least one population of immune cells,
wherein said exogenous cancer cells are present in at least one tissue of said embryo, and said exogenous immune cells are present in at least one tissue of said embryo and/or circulate in the blood vessels of said embryo,
wherein the gallinaceous bird embryo is from a chicken (*Gallus gallus*) or from a quail (*Coturnix japonica*)*,* and
wherein said embryo tissue designates any tissue of the embryo with the exception of the extra-embryonic annexes and the lumen of tubes and vessels.

2. Chimeric gallinaceous bird embryo according to claim 1, wherein the population of immune cells consists in Peripheral Blood Mononuclear Cells (PBMCs).

3. Chimeric gallinaceous bird embryo according to claim 1, wherein the population of immune cells consists in lymphocytes, in particular genetically engineered CAR-T cells.

4. Chimeric gallinaceous bird embryo according to anyone of claims 1 to 3, wherein cancer cells are issued from a tumor of a patient.

5. Chimeric gallinaceous bird embryo according to anyone of claims 1 to 4, wherein both populations of cells are human cells.

6. Chimeric gallinaceous bird embryo according to anyone of claims 1 to 5, wherein both types of exogenous cells are localized in the same at least one embryo tissue, in particular immune cells are present in the tumor(s) formed by cancer cells.

7. Chimeric gallinaceous bird embryo according to anyone of claims 1 to 6, wherein said at least one embryo tissue in which cancer cells are present is relevant with the nature of the population of cancer cells, in particular is representative of tissues/organs in which primary and/or secondary tumor(s) form(s) in cancer patients.

8. Chimeric gallinaceous bird embryo according to anyone of claims 1 to 7, wherein at least one population of exogenous cells is labelled.

9. Process for obtaining a chimeric gallinaceous bird embryo comprising both types of exogenous cells:
a. At least one population of cancer cells, and
b. At least one population of immune cells,
said process comprising the following steps:
• Introducing both populations of immune cells and cancer cells into a gallinaceous bird embryo, wherein the gallinaceous bird embryo is at a developmental stage between stage HH10 and stage HH30, and
• incubating the chimeric embryo for at least 6 hours at a temperature comprised between 35° C and 42° C,
wherein said exogenous cancer cells are introduced into at least one tissue of said embryo, and said exogenous immune cells are introduced in at least one tissue of said embryo and/or in the blood vessels of said embryo,
wherein the gallinaceous bird embryo is from a chicken *(Gallus gallus)* or from a quail *(Coturnix japonica),* and
wherein said embryo tissue designates any tissue of the embryo with the exception of the extra-embryonic annexes and the lumen of tubes and vessels.

10. Process according to claim 9, comprising the following steps:
• Co-grafting both populations of immune cells and cancer cells within at least one tissue of a gallinaceous bird embryo at a developmental stage between stage HH10 and stage HH30, and
• incubating the chimeric embryo for at least 6 hours at a temperature comprised between 35° C and 42° C.

11. Process according to claim 9, comprising the following steps:
• Grafting the at least one population of cancer cells within at least one tissue of a gallinaceous bird embryo at a developmental stage between stage HH10 and stage HH30,
• Injecting said at least one population of immune cells into the blood vessels of said embryo, and
• incubating the grafted embryo for at least 6 hours at a temperature comprised between 35° C and 42° C.

12. Animal model for the study of human cancers consisting in a chimeric gallinaceous bird embryo according to any one of claims 1 to 8.

13. Use of an animal model according to claim 12 for screening at least one anti-cancer therapy chosen among the following group: immunotherapy, chemotherapy, targeted therapy, hormonotherapy, anti-cancer drugs and therapeutic enzymes.

14. Use of an animal model according to claim 12 for assessing the activity of genetically engineered CAR-T cells.

## Patentansprüche

1. Chimärer Hühnervogelembryo, der beide Arten von exogenen Zellen enthält:
a. mindestens eine Population von Krebszellen, und
b. mindestens eine Population von Immunzellen,
wobei die exogenen Krebszellen in mindestens einem Gewebe des Embryos vorhanden sind und die exogenen Immunzellen in mindestens einem Gewebe des Embryos vorhanden sind und/oder in den Blutgefäßen des Embryos zirkulieren,
wobei der Hühnervogelembryo von einem Huhn (*Gallus gallus*) oder von einer Wachtel (*Coturnix japonica*) stammt, und
wobei das Embryogewebe jedes Gewebe des Embryos mit Ausnahme der extraembryonalen Anhänge und des Lumens von Tuben und Gefäßen bezeichnet.

2. Chimärer Hühnervogelembryo nach Anspruch 1, wobei die Population von Immunzellen aus mononukleären Zellen des peripheren Bluts (PBMC) besteht.

3. Chimärer Hühnervogelembryo nach Anspruch 1, wobei die Population der Immunzellen aus Lymphozyten, insbesondere gentechnisch veränderten CAR-T-Zellen, besteht.

4. Chimärer Hühnervogelembryo nach einem der Ansprüche 1 bis 3, wobei die Krebszellen aus einem Tumor eines Patienten stammen.

5. Chimärer Hühnervogelembryo nach einem der Ansprüche 1 bis 4, wobei beide Zellpopulationen menschliche Zellen sind.

6. Chimärer Hühnervogelembryo nach einem der Ansprüche 1 bis 5, wobei beide Arten von exogenen Zellen in demselben mindestens einen Embryogewebe lokalisiert sind, insbesondere Immunzellen in dem/den von Krebszellen gebildeten Tumor(en) vorhanden sind.

7. Chimärer Hühnervogelembryo nach einem der Ansprüche 1 bis 6, wobei das mindestens eine Embryogewebe, in dem Krebszellen vorhanden sind, für die Art der Krebszellenpopulation relevant ist, insbesondere repräsentativ für Gewebe/Organe ist, in denen sich bei Krebspatienten primäre und/oder sekundäre Tumore bilden.

8. Chimärer Hühnervogelembryo nach einem der Ansprüche 1 bis 7, wobei mindestens eine Population von exogenen Zellen markiert ist.

9. Verfahren zum Erhalten eines chimären Hühnervogelembryos, der beide Arten von exogenen Zellen enthält:
a. mindestens eine Population von Krebszellen, und
b. mindestens eine Population von Immunzellen,
wobei das Verfahren die folgenden Schritte umfasst:
• Einbringen beider Populationen von Immunzellen und von Krebszellen in einen Hühnervogelembryo, wobei sich der Hühnervogelembryo in einem Entwicklungsstadium zwischen Stadium HH10 und Stadium HH30 befindet, und
• Inkubieren des chimären Embryos für mindestens 6 Stunden bei einer Temperatur zwischen 35°C und 42°C,
wobei die exogenen Krebszellen in mindestens ein Gewebe des Embryos eingebracht werden und die exogenen Immunzellen in mindestens ein Gewebe des Embryos und/oder in die Blutgefäße des Embryos eingebracht werden,
wobei der Hühnervogelembryo von einem Huhn (*Gallus gallus*) oder von einer Wachtel (*Coturnix japonica*) stammt, und
wobei das Embryogewebe jedes Gewebe des Embryos mit Ausnahme der extraembryonalen Anhänge und des Lumens von Tuben und Gefäßen bezeichnet.

10. Verfahren nach Anspruch 9, das die folgenden Schritte umfasst:
• Co-Transplantieren beider Populationen von Immunzellen und von Krebszellen in mindestens ein Gewebe eines Hühnervogelembryos in einem Entwicklungsstadium zwischen Stadium HH10 und Stadium HH30, und
• Inkubieren des chimären Embryos für mindestens 6 Stunden bei einer Temperatur zwischen 35°C und 42°C.

11. Verfahren nach Anspruch 9, das die folgenden Schritte umfasst:
• Transplantieren der mindestens einen Population von Krebszellen in mindestens ein Gewebe eines Hühnervogelembryos in einem Entwicklungsstadium zwischen Stadium HH10 und Stadium HH30,
• Injizieren der mindestens einen Population von Immunzellen in die Blutgefäße des Embryos, und
• Inkubieren des transplantierten Embryos für mindestens 6 Stunden bei einer Temperatur zwischen 35°C und 42°C.

12. Tiermodell zur Untersuchung von menschlichen Krebsarten, bestehend aus einem chimären Hühnervogelembryo nach einem der Ansprüche 1 bis 8.

13. Verwendung eines Tiermodells nach Anspruch 12 für das Screening mindestens einer Krebstherapie, die ausgewählt ist aus der folgenden Gruppe: Immuntherapie, Chemotherapie, zielgerichtete Therapie, Hormontherapie, Krebsmedikamente und therapeutische Enzyme.

14. Verwendung eines Tiermodells nach Anspruch 12 zur Beurteilung der Aktivität von gentechnisch veränderten CAR-T-Zellen.

## Revendications

1. Embryon chimérique de gallinacé comprenant deux types de cellules exogènes :
a. Au moins une population de cellules cancéreuses, et
b. Au moins une population de cellules immunitaires,
dans lequel lesdites cellules cancéreuses exogènes sont présentes dans au moins un tissu embryonnaire, et lesdites cellules immunitaires exogènes sont présentes dans au moins un tissu embryonnaire et/ou circulent dans les vaisseaux sanguins dudit embryon,
**caractérisé en ce que** l'embryon de gallinacé est celui d'un poulet *(Gallus gallus*) ou d'une caille (*Coturnix japonica),* ledit tissu embryonnaire désignant tout tissu de l'embryon à l'exception des annexes extra-embryonnaires et de la lumière des tubes et des vaisseaux.

2. Embryon chimérique de gallinacé selon la revendication 1, dans lequel la population de cellules immunitaires est constituée de cellules mononucléaires du sang périphérique (PBMC).

3. Embryon chimérique de gallinacé selon la revendication 1, dans lequel la population de cellules immunitaires est constituée de lymphocytes, en particulier de cellules CAR-T génétiquement modifiées.

4. Embryon chimérique de gallinacé selon l'une des revendications 1 à 3, dans lequel les cellules cancéreuses proviennent d'une tumeur d'un patient.

5. Embryon chimérique de gallinacé selon l'une des revendications 1 à 4, dans lequel les deux populations de cellules sont des cellules humaines.

6. Embryon chimérique de gallinacé selon l'une des revendications 1 à 5, dans lequel les deux types de cellules exogènes sont localisés dans le même tissu embryonnaire, en particulier dans lequel les cellules immunitaires sont présentes dans la ou les tumeurs formées par des cellules cancéreuses.

7. Embryon chimérique de gallinacé selon l'une des revendications 1 à 6, dans lequel ledit au moins un tissu embryonnaire dans lequel des cellules cancéreuses sont présentes est corrélé avec la nature de la population de cellules cancéreuses, notamment qui est représentatif des tissus/organes dans lesquels se forment des tumeurs primaires et/ou secondaires chez les patients atteints de cancer.

8. Embryon chimérique de gallinacé selon l'une des revendications 1 à 7, dans lequel au moins une population de cellules exogènes est marquée.

9. Procédé d'obtention d'un embryon chimérique de gallinacé comprenant deux types de cellules exogènes :
• Au moins une population de cellules cancéreuses, et
• Au moins une population de cellules immunitaires,
ce procédé comprenant les étapes suivantes :
• introduction de deux populations de cellules immunitaires et cancéreuses dans un embryon de gallinacé, ledit embryon étant à un stade de développement compris entre le stade HH10 et le stade HH30, et
• incubation de l'embryon chimérique pendant au moins 6 heures à une température comprise entre 35° C et 42° C,
**caractérisé en ce que** lesdites cellules cancéreuses exogènes sont introduites dans au moins un tissu embryonnaire, et lesdites cellules immunitaires exogènes sont introduites dans au moins un tissu embryonnaire et/ou dans les vaisseaux sanguins dudit embryon,
et **caractérisé en ce que** l'embryon de gallinacé est celui d'un poulet *(Gallus gallus*) ou d'une caille (*Coturnix japonica),*
ledit tissu embryonnaire désignant tout tissu de l'embryon à l'exception des annexes extra-embryonnaires et de la lumière des tubes et des vaisseaux.

10. Procédé selon la revendication 9, comprenant les étapes suivantes :
• co-greffe des deux populations de cellules immunitaires et cancéreuses dans au moins un tissu d'un embryon de gallinacé à un stade de développement compris entre le stade HH10 et le stade HH30, et
• incubation de l'embryon chimérique pendant au moins 6 heures à une température comprise entre 35° C et 42° C.

11. Procédé selon la revendication 9, comprenant les étapes suivantes :
• Greffe d'au moins une population de cellules cancéreuses dans au moins un tissu d'un embryon de gallinacé à un stade de développement compris entre le stade HH10 et le stade HH30,
• Injection de ladite au moins une population de cellules immunitaires dans les vaisseaux sanguins dudit embryon, et
• incubation de l'embryon greffé pendant au moins 6 heures à une température comprise entre 35° C et 42° C.

12. Modèle animal pour l'étude des cancers humains consistant en un embryon chimérique de gallinacé selon l'une des revendications 1 à 8.

13. Utilisation d'un modèle animal selon la revendication 12 pour le criblage d'au moins une thérapie anticancéreuse choisie parmi le groupe suivant : immunothérapie, chimiothérapie, thérapie ciblée, hormonothérapie, médicaments anticancéreux et enzymes thérapeutiques.

14. Utilisation d'un modèle animal selon la revendication 12 pour évaluer l'activité de cellules CAR-T génétiquement modifiées.
